# EUROPÄISCHE PATENTSCHRIFT

(11) **EP 1 749 810 B1**
(45) Veröffentlichungstag und Bekanntmachung des Hinweises auf die Patenterteilung: **24.12.2008**
(21) Anmeldenummer: 06117893.5
(22) Anmeldetag: 26.07.2006
(51) Int. Cl.: C07C 29/17, C07C 31/135

(54) **Verfahren zur Herstellung von 1-(2,2,6-Trimethylcyclohexyl)-hexan-3-ol**
Process for the preparation of 1-(2,2,6-trimethylcyclohexyl)-hexan-3-ol
Procédé de préparation de 1-(2,2,6-trimethylcyclohexyl)-hexan-3-ol

(30) Priorität: 04.08.2005 DE 102005036672
(43) Veröffentlichungstag der Anmeldung: 07.02.2007
(73) Patentinhaber: Symrise GmbH & Co. KG, 37603 Holzminden (DE)
(72) Erfinder: Schatkowski, Dietmar, 37627, Stadtoldendorf (DE)
(74) Vertreter: Eisenführ, Speiser & Partner

(56) Entgegenhaltungen:
- DE-A1- 10 062 771

## Beschreibung

Die vorliegende Erfindung betrifft ein Verfahren zur Herstellung von 1-(2,2,6-Trimethylcyclohexyl)-hexan-3-ol der Formel A 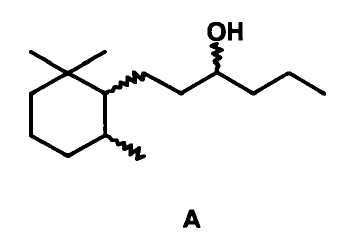 mit einem Anteil von zumindest 30% Gewichtsprozent des entsprechenden trans-Isomeren der Formel B, bezogen auf die Gesamtmenge 1-(2,2,6-Trimethylcyclohexyl)-hexan-3-ol der Formel A. Neben dem trans-Isomeren liegt dabei das cis-isomere der Formel C vor.

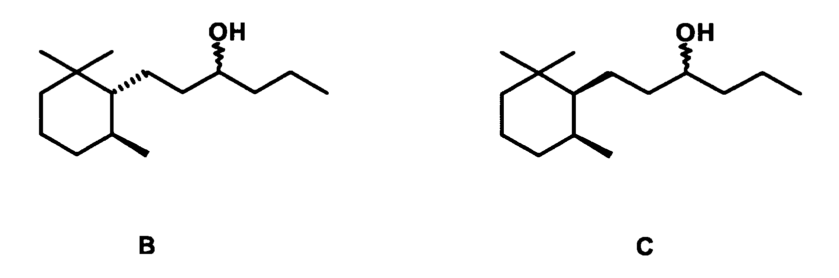

Die Erfindung betrifft zudem ein Verfahren zur Herstellung einer entsprechenden Parfümkomposition.

1-(2,2,6-Trimethylcyclohexyl)-hexan-3-ol der Formel A (umfassend die Isomeren der Formeln B und C) ist eine Verbindung, die wegen ihres charakteristischen, holzigen/ambrierten Geruchs sowie der guten fixierenden Eigenschaften und Wirkungen eine bereite Verwendung in der Herstellung von Parfümkompositionen gefunden hat, vgl. DE 24 55 761 und DE 28 07 584.

Die Verbindung der Formel A lässt sich z.B. über folgenden Reaktionsweg 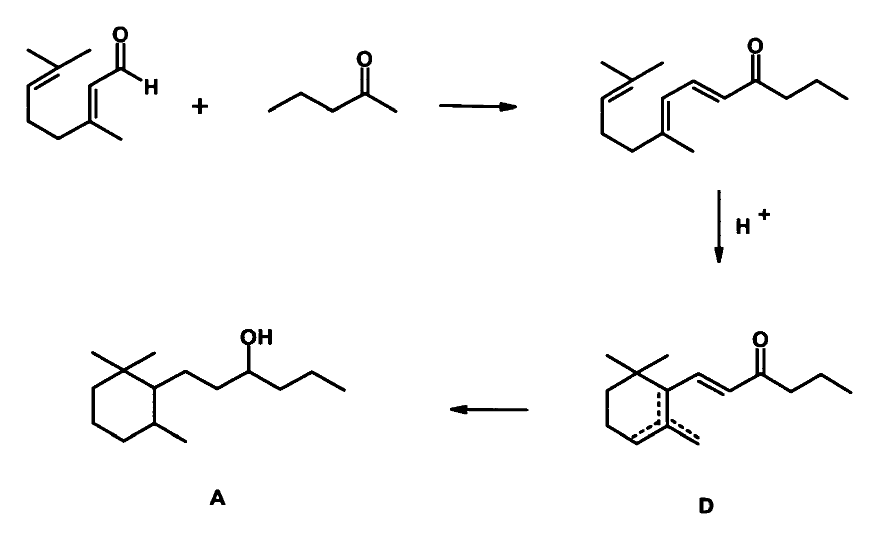 herstellen:

Die in Formel D eingezeichneten gestrichelten Linien stehen dabei für eine einzelne Doppelbindung, die in einer der drei eingezeichneten Positionen angeordnet sein kann. Unter Verwendung der für Jonone üblichen Nomenklatur handelt es sich also um eine α-,β- oder γ-ständige Doppelbindung (vergleiche Römpp-Lexikon "Naturstoffe", Thieme Verlag, 1997, Seite 334 - 335).

Die Verbindungen der Formel D sind dabei durch dem Fachmann geläufige Reaktionen, wie z.B. durch Kondensation von Citral mit 2-Pentanon und anschließende säurekatalysierte Cyclisierung herstellbar; vergleiche Helv. Chim.Acta 1948, 31, 417.

DE 24 55 761 offenbart, dass bei der Hydrierung von z.B. Methyljononen in Gegenwart von Raney-Nickel als alleinigem Katalysator nur 12% der theoretisch möglichen Menge an Wasserstoff aufgenommen werden. Gemäß der DE 24 55 761 führt eine Hydrierung in Gegenwart von Raney-Nickel allein nicht zur Reduktion der Carbonylgruppe, sondern nur bei gleichzeitiger Gegenwart von Raney-Nickel und Kupferchromit.

EP 1 400 503 offenbart, dass insbesondere unter Zusatz einer Base und unter Einhaltung bestimmter Temperatur- und Druckbereiche eine vollständige Hydrierung mit Raney-Nickel auch in Abwesenheit von Kupferchromit gelingt, wobei mindestens 15% der sensorisch wertvolleren trans-Isomeren der Formel B entstehen.

DE 100 62 771 offenbart zwei alternative Verfahrensvarianten. Gemäß einer ersten Verfahrensvariante werden 1-(2,2,6-Trimethyl-1- bzw. -2-cyclohexen-1-yl)-1-alken-3-one unter Verwendung von Ruthenium-Katalysatoren zu den entsprechenden 1-(2,2,6-Trimethylcyclohexyl)-3-alkanolen mit einem Gehalt von 20% an trans-Isomeren reduziert. Die Reaktion wird dabei bevorzugt bei einem Wasserstoffdruck im Bereich von 10 bis 20 bar und einer Temperatur von 130°C bis 150°C durchgeführt. Nachteilig bei dieser ersten Verfahrensvariante gemäß DE 100 62 771 ist jedoch die für eine industrielle Umsetzung sehr langsame Reaktionszeit von 60 Stunden.

Gemäß der zweiten in DE 100 62 771 offenbarten Verfahrensvariante werden zunächst durch eine technisch aufwendige Reduktion von 1-(2,2,6-Trimethyl-1- bzw. -2-cyclohexen-1-yl)-1-alken-3-onen mit Natriumborhydrid 1-(2,2,6-Trimethylcyclohex2-enyl)-alk-1-en-3-ole hergestellt, welche anschließend in Gegenwart von Hydrier-Katalysatoren auf Basis von Elementen der Nebengruppen Ib, Vlb und Vlllb hydriert werden. Unter anderem wird Rhodium als einsetzbarer Hydrier-Katalysator genannt, und besonders bevorzugt wird bei einem Wasserstoffdruck im Bereich von 5 bis 15 bar und einer Temperatur im Bereich von 20°C bis 50°C hydriert, wobei bevorzugt ein Gehalt an trans-Isomeren im Bereich zwischen 30% und 40% erhalten wird.

In Helv. Chim. Acta 1999, 82, 1762-1773 ist die Hydrierung von 1-(2,6,6-Trimethylcyclohex-2-enyl)-hex-1-en-3-olen unter Verwendung von Pd/C bzw. PtO₂ beschrieben. Bei Raumtemperatur wurden in Gegenwart von 30 Gew.-% Pd/C (10%ig auf Aktivkohle) in Essigsäure bei einem Druck von 3 bar 1-(2,2,6-Trimethylcyclohexyl)-hexan-3-ole der Formel A erhalten, wobei das Verhältnis der trans-Isomeren der Formel B zu den cis-Isomeren der Formel C bei 40 : 60 lag.

Nach P. N. Rylander [Catalytic Hydrogenation in Organic Synthesis, Academic Press 1979, ISBN 0-12-605355-3] unterscheiden sich die Hydrierkatalysatoren in einem weiten Bereich hinsichtlich Aktivität und Selektivität. Die Elemente der Gruppe Vlllb gelten allgemein als Katalysatoren für den Niederdruck-Bereich. So wird beispielsweise in Adv. Catal., 9, 773, (1957) auf die bei niedrigen Temperaturen und Drücken ausreichende Aktivität für die Durchführung von Hydrierungen hingewiesen.

In J. Amer. Chem. Soc. 1962, 84, 3132-3136 wurde die katalytische Hydrierung von 4-tert.-Butyl-1-methylencyclohexan untersucht. Wird die Hydrierung von 4-tert.-Butyl-1-methylencyclohexan bei niedrigen Drücken mit Palladium durchgeführt, so wird im Vergleich zu mit Platinoxid gefundenen Ergebnissen mehr trans-Verbindung erhalten. Bei Einsatz von Rhodium bzw. Ruthenium als Hydrier-Katalysatoren wurde im Vergleich zu den Verfahrensgestaltungen mit Einsatz von Platinoxid oder Palladium jeweils deutlich mehr von der entsprechenden cis-Verbindung erhalten.

Diese und andere Arbeiten zeigen, dass Produktzusammensetzungen nach Hydrierungen in Gegenwart von Hydrier-Katalysatoren mit Elementen der Nebengruppe Vlllb in Abhängigkeit von den Reaktionsbedingungen deutliche Unterschiede aufweisen und generelle Vorhersagen zu den zu erwartenden stereochemischen Produktzusammensetzungen nicht getroffen werden können.

Weitere Veröffentlichungen beschreiben die Herstellung von Verbindungen der Formel A mit einem möglichst hohen Anteil an trans-Isomeren der Formel B. Insbesondere sei insoweit auf die EP 0 118 817 und die EP 0 456 932 verwiesen. Die darin beschriebenen Verfahren sind jedoch entweder aufgrund der Verwendung schwierig zu handhabender Reagenzien, wie z.B. Lithiumaluminiumhydrid oder Natriumborhydrid, oder aufgrund ihrer Vielstufigkeit für einen technischen Maßstab wenig geeignet.

Wie bereits in EP 0 118 817 A1 offenbart, zeichnen sich die trans-Verbindung der Formel B und die isomere cis-Verbindung der Formel C dadurch aus, dass sie unterschiedliche Geruchsprofile besitzen. EP 0 118 817 A1 beschreibt Isomerengemische mit einem Anteil an trans-1-(2,6,6-Trimethylcyclohexyl)-hexan-3-olen der Formel B von mindestens 60% und einem Anteil an cis 1-(2,6,6-Trimethylcyclohexyl)-hexan-3-olen der Formel C von nicht mehr als 40%.

In "Riechstoffe und Geruchssinn" (Springer Verlag Heidelberg 1990; ISBN 3-540-52560) werden die isomeren Verbindungen geruchlich folgendermaßen beschrieben: die cis-1-(2,6,6-Trimethylcyclohexyl)-hexan-3-ole der Formel C riechen holzig-ambriert, gepaart mit einer schweißigen Nuance. Die trans-1-(2,6,6-Trimethylcyclohexyl)-hexan-3-ole der Formel B besitzen einen ausgesprochen animalischen und Ambracharakter, wobei der animalischen Note schon deutlich unangenehme fekalische, kotige Geruchseindrücke zugeschrieben werden. Schweißige (cis) bzw. fekalische (trans)-Geruchseindrücke sind in der Parfümierung von Handelsproduktion jedoch unerwünscht.

EP 0 118 809 A2 beschreibt Mischungen von 1-(2,6,6-Trimethylcyclohexyl)-hexan-3-olen enthaltend mindestens 50 Gew.-% der trans-Isomeren B und höchstens 50 Gew.-% der cis-Isomeren C als geeignet für die meisten Gebiete der Parfümerie.

Zudem verwiesen sei auf die folgenden Dokumente: Houben-Weyl, Methoden der org. Chemie, Band IV/1c, Seite 192-193 sowie E. Breitner, E. Roginski & P.N. Rylander, J. Org. Chem. 24, 1855 (1959).Veränderungen in der Isomerenzusammensetzung hin zu einem geringeren trans-Anteil als 50% Gew.-% führen zu deutlich holzigeren Geruchseindrücken mit einem Anklang an Vetiveröl. Das unter dem Produktnamen Timberol vertriebene Produkt, mit einem Anteil von trans-Isomer der Formel B von 10-12% wird sogar mit "cedernholzartig" beschrieben. Dieser Geruchseindruck lässt das Produkt parfümistisch eher minderwertig erscheinen, da Cedernholzöl in großen Mengen verfügbar ist und für die Parfümierung preiswerter Handelsprodukte in großen Mengen eingesetzt wird.

Es war eine Aufgabe der vorliegenden Erfindung, ein Verfahren zur Herstellung von 1-(2,6,6-Trimethylcycloheyl)-hexan-3-olen der Formel A anzugeben, wobei vorzugsweise ein Isomeren-Gemisch hergestellt werden sollte, welche weder die negativen fekalischen Geruchseindrücke der reinen trans-Isomere der Formel B noch die schweißigen Geruchseindrücke der cis-Isomere der Formel C in störendem Maße aufweist.

Vorzugsweise sollte das anzugebende Herstellverfahren von einfach zugänglichen und preiswerten Edukten ausgehen und in nur einem Reaktionsschritt und mit einer vergleichsweisen kurzen Reaktionszeit das gewünschte Produkt liefern, so dass das Verfahren auch für den technischen Maßstab geeignet ist.

Schließlich sollte das anzugebende Verfahren auch einen hohen Umsatz der einzusetzenden Edukte ermöglichen, um auf diese Weise vergleichsweise reine Produktgemische erzielen zu können.

Die gestellte Aufgabe wird erfindungsgemäß gelöst durch ein Verfahren zur Herstellung von 1-(2,2,6-Trimethylcyclohexyl)-hexan-3-ol der Formel A 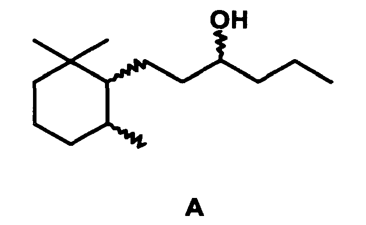 mit einem Anteil von zumindest 30 Gew.-% des entsprechenden trans-Isomeren, bezogen auf die Gesamtmenge 1-(2,2,6-Trimethylcyclohexyl)-hexan-3-ol der Formel A, mit folgendem Schritt:
Katalytisches Hydrieren einer Verbindung der Formel D, 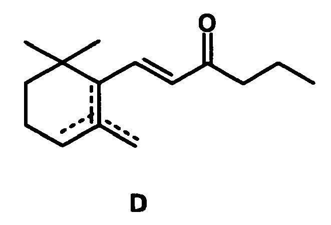 in der die gestrichelten Linien für eine einzelne Doppelbindung stehen, die in einer der drei eingezeichneten Positionen angeordnet ist, in Gegenwart eines Rhodium-Katalysators zum 1-(2,2,6-Trimethylcyclohexyl)-hexan-3-ol der Formel A.

Formel D umfasst die folgenden Verbindungen:

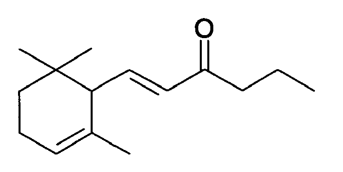

### (1E)-1-(2,6,6-trimethylcyclohex-2-en-1-yl)hex-1-en-3-on

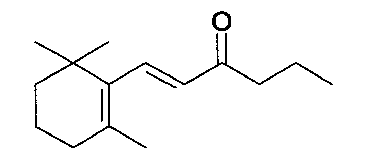

### (1E)-1-(2,6,6-trimethylcyclohex-1-en-1-yl)hex-1-en-3-on

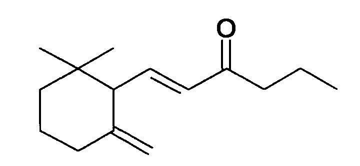

### (1E)-1-(2,2-dimethyl-6-methylenecyclohexyl)hex-1-en-3-on

Das erfindungsgemäße Verfahren ermöglicht auf einfache Weise die Herstellung von 1-(2,6,6-Trimethylcyclohexyl)-hexan-3-olen der Formel A, wobei der Anteil an trans-Isomeren der Formel B im Reaktionsprodukt vorzugsweise sogar im Bereich von 40 Gew.-% bis 50 Gew.-% liegt, bezogen auf die Gesamtmenge 1-(2,6,6-Trimethylcyclohexyl)-hexan-3-ol der Formel A, abhängig von der Wahl der Reaktionsbedindungen. Der Anteil des cis-Isomeren liegt dementsprechend vorzugsweise im Bereich von 60 Gew.-% bis 50% Gew.-%. Die Einstellung des entsprechenden trans-/cis-Isomerenverhältnisses kann erfindungsgemäß (a) ohne einen zusätzlichen (aufwendigen) Destillationsschritt und (b) ohne vorherige Reduktion der Carbonylgruppe der als Edukt eingesetzten Verbindung der Formel D (unter Verwendung von gemäß dem Stand der Technik einzusetzenden Reagenzien, wie z.B. Lithiumaluminiumhydrid oder Natriumborhydrid) erhalten werden. Dies unterscheidet das erfindungsgemäße Verfahren deutlich von den Verfahrensvarianten gemäß DE 100 62 771 A1.

Vorzugsweise wird in dem erfindungsgemäßen Verfahren die Hydrierung erst nach Umsetzung von zumindest 98 % der Verbindung der Formel D beendet.

Im erfindungsgemäßen Verfahren wird die Hydrierung in einem Wasserstoff-Druckbereich von 80 - 200 bar durchgeführt. Die Einstellung eines Wasserstoffdrucks in einem Bereich von 80 - 200 bar sowie in einem bevorzugten Bereich von 80 - 150 bar ermöglicht auf besonders einfache Weise das Erreichen eines Anteils von 40 Gew.-% bis 50 Gew.-% des trans-Isomeren im hergestellten Isomeren-Produktgemisch, hierbei beziehen sich die Prozentangaben wieder auf die Gesamtmenge des hergestellten 1-(2,6,6-Trimethylcyclohexyl)-hexan-3-ols der Formel A. Ein Wasserstoffdruck im Bereich von über 100 bar bis 200 bar ist besonders bevorzugt. Ganz besonders bevorzugt ist der Bereich von über 100 bar bis 150 bar. Insbesondere in der Auswahl der bevorzugten Wasserstoffdruck-Bereiche (in Kombination mit der Auswahl des Katalysators) ist ein weiterer bedeutender Unterschied zum bekannten Stand der Technik zu sehen.

In dem erfindungsgemäßen Verfahren wird die katalytische Hydrierung bei einer Temperatur im Bereich von 180°C bis 260°C durchgeführt. Hinsichtlich der bevorzugten Wasserstoff-Druckbereiche gilt dabei das zuvor Gesagte.

Im erfindungsgemäßen Verfahren wird ein Rhodium-Katalysator eingesetzt. Vorzugsweise wird dabei Rhodium in einer Menge von 0,0001 bis 10 Gew.-% eingesetzt, vorzugsweise im Bereich von 0,01 bis 0,5 Gew.-%, bezogen auf die Menge der eingesetzten Verbindung der Formel D.

Das als Katalysator verwendete Rhodium ist vorzugsweise auf ein Trägermaterial aufgebracht. Bevorzugt sind Trägermaterialien wie Aktivkohle, Aluminiumoxid oder Siliciumoxid. Die Menge des Rhodiumkatalysators auf dem Trägermaterial liegt bevorzugt im Bereich von 5 bis 10 Gew.-%, bezogen auf die Gesamtmasse von Trägermaterial und Rhodium.

In eigenen Untersuchungen wurden besonders gute Resultate erzielt bei Verwendung von Rhodium-Katalysatoren auf Aktivkohle oder einem anderen Trägermaterial wie Aluminiumoxid in einer Menge von 0,0001 bis 10 Gew.-%, vorzugsweise von 0,01 bis 0,5 Gew.-%, bezogen auf die Menge der eingesetzten Verbindung der Formel D. Höhere Katalysatormengen führen dabei regelmäßig zu vergleichsweise hohen Anteilen an trans-Isomeren (Formel B) und ermöglichen kurze Reaktionszeiten (vgl. die Beispiele 1-3, unten).

Das erfindungsgemäße Verfahren kann wahlweise kontinuierlich und diskontinuierlich durchgeführt werden.

Im Falle einer diskontinuierlichen Reaktionsführung (Batch) wird der Rhodium-Katalysator bevorzugt als Pulver eingesetzt.

Auf Grund der kurzen Reaktionszeiten kann das erfindungsgemäße Verfahren auch in einem Festbettreaktor durchgeführt werden, wie beispielsweise einem Rieselbettreaktor, an einem Katalysator-Festbett. Im Falle einer kontinuierlichen Reaktionsführung ist es in manchen Fällen vorteilhaft, den Rhodium-Katalysator als Formkörper oder Hohlkörper zu verwenden. Hierbei können insbesondere Körper in der Gestalt von Hohlsträngen, Extrudaten, Pellets, Strangpresslingen, Wirbelsträngen, Sätteln, Ringen, Hohlkugeln, Kugeln, Hohlzylindern, Zylindern, Würfeln, Tabletten, Kegeln und dergleichen eingesetzt werden.

In einem erfindungsgemäßen Verfahren kann der Schritt des katalytischen Hydrierens sowohl in Substanz (d.h. lösungsmittelfrei) als auch in Lösung durchgeführt werden. Als Lösungsmittel für die Edukt-Verbindung der Formel D und die Produkt-Verbindung der Formel A eignen sich Stoffe wie Alkohole (z.B. Methanol, Ethanol, Isopropanol), Ester (z.B. Ethylacetat) oder Kohlenwasserstoffe (z.B. Hexan oder Cyclohexan) sowie deren Mischungen.

Das erfindungsgemäße Verfahren unter Verwendung von Rhodium-Katalysatoren führt ausgehend von einer Verbindung der Formel D zu einem Reaktionsprodukt der Formel A mit einem hohen Anteil an trans-Isomeren der Formel B, und zwar in einem einzigen Reaktionsschritt ohne eine Vorab-Reduktion (Hydrierung) der Oxo-Gruppe. Die Edukt-Verbindungen der Formel D sind leicht zugängliche Verbindungen, so dass das erfindungsgemäße Verfahren insgesamt mit einem nur geringen technischen Aufwand durchführbar ist. Das erfindungsgemäße Verfahren führt zudem zu einer besonders vollständigen Umsetzung der Edukt-Verbindungen der Formel D. Ein Umsetzungsgrad > 98% ist überraschend leicht erreichbar. Die vollständige Umsetzung führt letztlich zu einer verbesserten Qualität der Produkte und erleichtert deren Aufreinigung.

Gemäß einer bevorzugten Ausgestaltung wird in einem erfindungsgemäßen Verfahren
(a) das hergestellte 1-(2,2,6-Trimethylcyclohexyl)-hexan-3-ol der Formel A
   (i) von dem Rhodium-Katalysator und/oder
   (ii) von sonstigen Bestandteilen des Produktgemisches abgetrennt und/oder
(b) ein Isomeres des hergestellten 1-(2,2,6-Trimethylcyclohexyl)-hexan-3-ols der Formel A aus dem Produktgemisch abgetrennt oder aufkonzentriert.

Die vorliegende Erfindung betrifft auch ein Verfahren zur Herstellung einer Parfümkomposition, mit folgenden Schritten:
- Herstellen von 1-(2,2,6-Trimethylcyclohexyl)-hexan-3-ol gemäß einem erfindungsgemäßen Verfahren (wie vorstehend beschrieben).
- Gegebenenfalls Isolieren des 1-(2,2,6-Trimethylcyclohexyl)-hexan-3-ols aus dem Produktgemisch und/oder Reinigen des Produktgemisches,
und nachfolgend
- Vermischen einer sensorisch wirksamen Menge des 1-(2,2,6-Trimethylcyclohexyl)-hexan-3-ols mit einem oder mehreren weiteren Riechstoffen.

Hinsichtlich bevorzugter Ausgestaltungen bei der Herstellung des 1-(2,2,6-Trimethylcyclohexyl)-hexan-3-ol gilt dabei natürlich das zuvor Gesagte.

In einer erfindungsgemäß hergestellten Parfümkomposition liegt der Anteil an 1-(2,2,6-Trimethylcyclohexyl)-hexan-3-ol der Formel A vorzugsweise im Bereich von 0,0001 bis 50 Gew.-%, bevorzugt im Bereich von 0,01 bis 20 Gew.-%, bezogen auf das Gesamtgewicht der Parfümkomposition. Vorzugsweise liegt hierbei der Anteil von trans-Isomeren der Formel B im Bereich von 40 bis 50 Gew.-%, bezogen auf das Gesamtgewicht der Trimethylcyclohexyl-hexan-3-ol der Formel A.

Nachfolgend wird die Erfindung anhand von Beispielen weiter erläutert. In den Beispielen beziehen sich alle Angaben auf das Gewicht, sofern nicht andern angegeben. Die Abkürzung DPG steht für Dipropylenglycol.

### Beispiel 1 (erfindungsgemäß):

### Herstellung von 1-(2,2,6-Trimethylcycloheyl)-hexan-3-ol.

In einem Rührautoklaven mit Begasungsrührer wurden 2000 g eines Gemisches aus 1-(2,6,6-Trimethylcyclohex-1-en-1-yl-oder -2-en-1-yl-)-hex-1-en-3-on (gemäß GC ca. 80%ig) mit 0,4 g Rhodium 10 % auf Kohle, entsprechend 0,02 Gew.- % Rhodium auf Aktivkohle, bei 100 bar und einer Reaktionstemperatur von 200°C - 220 °C während 2 Stunden hydriert. Die Aufheizung erfolgte innerhalb von 45 Minuten. Nach Filtration und Destillation wurden 1560 g vollständig hydriertes Produkt erhalten. Das Gewichtsverhältnis von trans-1-(2,2,6-Trimethylcyclohexyl)-hexan-3-ol der Formel B zu cis-1-(2,2,6-Trimethylcyclohexyl)-hexan-3-ol der Formel C lag bei 40 : 60.

### Beispiel 2 (erfindungsgemäß)

### Herstellung von 1-(2,2,6-Trimethylcycloheyl)-hexan-3-ol.

In einem Rührautoklaven mit Begasungsrührer wurden 2000 g eines Gemisches aus 1-(2,6,6-Trimethylcyclohex-1-en oder 2-en-1-yl)-hex-1-en-3-on (gemäß GC ca. 80%ig) mit 1 g Rhodium 10 % auf Aktivkohle, entsprechend 0,05 Gew.-% Rhodium auf Aktivkohle, bei 100 bar und einer Temperatur von 180 °C bis 260 °C während 1 Stunde hydriert. Die Aufheizung erfolgte dabei innerhalb von 30 Minuten. Nach Filtration und Destillation wurde vollständig hydriertes Produkt erhalten (M 2; vgl. Beispiel 7). Das Gewichtsverhältnis von trans-1-(2,2,6-Trimethylcyclohexyl)-hexan-3-ol der Formel B zu cis-1-(2,2,6-Trimethylcyclohexyl)-hexan-3-ol der Formel C lag bei 49 : 51.

### Beispiel 3 (erfindungsgemäß):

### Herstellung von 1-(2,2,6-Trimethylcycloheyl)-hexan-3-ol.

In einem Rührautoklaven mit Begasungsrührer wurden 2000 g eines Gemisches aus 1-(2,6,6-Trimethylcyclohex-1-en oder 2-en-1-yl)-hex-1-en-3-on (gemäß GC ca. 80 %ig) mit 5 g Rhodium 5% auf aktiviertem Aluminiumoxid, entsprechend 0,25 Gew.-% Rhodium auf Aluminiumoxid, bei 130 bar und einer Temperatur von 220°C bis 260 °C während 1 Stunde hydriert. Die Aufheizung erfolgte dabei innerhalb von 40 Minuten. Nach Filtration und Destillation wurde vollständig hydriertes Produkt erhalten. Das Verhältnis trans-/cis-1-(2,2,6-Trimethylcyclohxeyl)hexan-3-ol betrug 50 : 50.

### Beispiel 4 (nicht erfindungsgemäß)

### Herstellung von 1-(2,2,6-Trimethylcycloheyl)-hexan-3-ol in Gegenwart eines Raney-Nickelkatalysators.

In einem Rührautoklaven mit Begasungsrührer wurden 2000 g eines Gemisches aus 1-(2,6,6-Trimethylcyclohex-1-en oder 2-en-1-yl)-hex-1-en-3-on (nach GC ca. 80%ig) mit 60 g Raney Nickel, entsprechend 3 Gew.-% Raney Nickel, bei 40 bar Wasserstoffdruck und einer Reaktionstemperatur von 280°C bis 300°C während einer Stunde hydriert. Die Aufheizung auf Reaktionstemperatur erfolgte dabei innerhalb von 50 Minuten. Nach Filtration und Destillation wurde das vollständig hydrierte Produkt erhalten. Das Verhältnis von trans- : cis-1-(2,2,6-Trimethylcyclohexyl)-hexan-3-ol lag bei 1: 5 (= 16, 6 : 83,3).

### Beispiel 5

### Gewinnung von reinen cis-1-(2,2,6-Trimethylcyclohexyl)-hexan-3-olen der Formel C.

50 g feindestilliertes Alkoholgemisch aus Beispiel 4, welches die beiden Alkohole in einem trans/cis - Verhältnis von 1 : 5 enthält, wurden an einer Fischer Spaltrohr-Kolonne nochmals feindestilliert. Dabei wurden 25 g cis-1-(2,2,6-Trimethylcyclohexyl)-hexan-3-ol der Formel C in 96%iger Reinheit mit einem Restgehalt von 3% trans-1-(2,2,6-Trimethylcyclohexyl)-hexan-3-ol der Formel B als farbloses Öl erhalten (M 1; vgl. Beispiel 7).
Kp_{1mbar}138 °C - 139°C

### Beispiel 6

40 g feindestilliertes Alkoholgemisch aus Beispiel 2 wurden an einer Fischer Spaltrohr - Kolonne nochmals feindestilliert. Es wurden 5 g trans-1-(2,2,6-Trimethylcyclohexyl)-hexan-3-ol der Formel B in 97%iger Reinheit mit einem Restgehalt von 2% cis-1-(2,2,6-Trimethylcyclohexyl)-hexan-3-olen der Formel C als farbloses Öl erhalten (M 3; vgl. Beispiel 7).
Kp_{1mbar} 140°C - 141°C

### Beispiel 7:

### Sensorische Vergleichsuntersuchung: Mischungen mit unterschiedlichen Anteilen des cis- und/oder trans-Isomeren.

Zunächst erfolgte eine sensorische Bewertung dreier Proben M 1, M 2 und M 3:
M 1 aus Beispiel 5
   96 % cis 1-(2,2,6-Trimethylcyclohexyl)hexan-3-ol
M 2 aus Beispiel 2
   49 % trans 1-(2,2,6-Trimethylcyclohexyl)hexan-3-ol
   51 % cis 1-(2,2,6-Trimethylcyclohexyl)hexan-3-ol
M 3 aus Beispiel 6
   97 % trans 1-(2,2,6-Trimethylcyclohexyl)hexan-3-ol

Die sensorische Beurteilung der drei Proben M 1, M 2 und M 3 wurde mit einem Expertenpanel aus acht Personen (vier Frauen und vier Männer) durchgeführt. Die Proben wurden jeweils als 5 Gew.-%ige ethanolische Lösung nach dem weitgehenden Verdunsten des Ethanols am Riechstreifen wie folgt beurteilt:
M 1: 96 % cis-1-(2,2,6-Trimethylcyclohexyl)hexan-3-ol der Formel C

Diffuse, animalisch, schweißig mit einem leicht ranzigen Unterton, schwach ambriert mit Anklänge an Schwertlilien, die weiblichen Personen fanden auch deutlich urinige Anklänge.
M 2: Mischung von trans- und cis-1-(2,2,6-Trimethylcyclohexyl)-hexan-3-olen aus Beispiel 2.

Schwer, betäubend, narkotisch, starke ambrierte Note, diffuse, sehr intensive Ausstrahlung mit einer Note, welche an natürliche Ambra erinnert, sehr ausgewogener Gesamteindruck.
M 3: 97 % trans-1-(2,2,6-Trimethylcyclohexyl)hexan- 3- ol der Formel B

Animalisch, fekalischer Gesamteindruck, welcher sich mit zunehmender Trocknungszeit verstärkt, leicht ambriert.

Alle Proben verfügen über einen starken Nachgeruch, der mehrere Wochen anhält, wobei sich insgesamt die erfindungsgemäß hergestellte Probe M 2 durch den ausgewogensten und harmonischsten Gesamteindruck auszeichnete und die höchste Geruchsintensität der drei Proben aufwies. Bei den Proben M 1 und M 3 verstärkten sich nach mehreren Tagen die oben bereits beschriebenen negativen Geruchseindrücke.

Die Proben M 1, M 2 und M 3 wurden im nachfolgenden Beispiel 8 eingesetzt.

### Beispiel 8

### Einfluss des Mischungsverhältnisses der cis/trans-Isomeren auf den sensorischen Eindruck verschiedener Parfümbasen.

Es wurden die folgenden Parfümbasen hergestellt:

### 8.1 1 Parfümbase des blumig-holzigen Typs

| | |
|---|---|
| Bergamotte Öl | 7,5 |
| Linalool | 4,0 |
| Phenylethylalkohol | 5,0 |
| Benzyl acetat | 2,0 |
| Citronellol | 2,0 |
| Hedione (a) | 10,0 |
| Ysamber - K^{®} (c) | 5,0 |
| Lyral (b) | 4,0 |
| Hydroxicitronellal | 2,5 |
| Rosenoxid (c), 10 % in DPG | 2,5 |
| Hexylzimtaldehyd, alpha | 7,5 |
| Patchouli Öl indonesisch | 4,0 |
| Iso-E-Super (b) | 2,0 |
| Vetiverylacetat | 2,0 |
| Brahmanol F (c) | 2,0 |
| Benzylsalicylat | 2,0 |
| Cis-3-Hexenylsalicylat | 1,0 |
| Cedramber (b) | 1,0 |
| Ambrettolide | 1,0 |
| Indol, 10 % in DPG | 0,5 |
| Opoponax Extrakt | 0,5 |
| Eichenmossextrakt, 50 % in DPG | 5,0 |
| Lilial | 10,0 |
| Total | 83,0 |

(a) Firmenich
(b) IFF
(c) Symrise

Die Parfümbase der angegebenen Formel besitzt einen ausgewogenen blumigen-holzigen Duftcharakter.

### 8.2 Parfümbase vom Fougere-Typ

| | |
|---|---|
| Bergamotte Öl | 18,0 |
| Lavandinöl Super | 15,0 |
| Lilial | 10,0 |
| Anisaldehyd | 3,0 |
| Coumarin | 5,0 |
| Hexylzimtaldehyd, alpha | 20,0 |
| Ambrinolepoxid, 10 % in DPG | 0,5 |
| Ambroxid (c) | 1,0 |
| Cantryl (c) | 10,0 |
| Pefferminzöl | 1,0 |
| Total | 83,5 |

Die Parfümbase der angegebenen Formel besitzt einen frisch - krautigen Fougere Duft.

### 8.3 Parfümbase vom grün-blumigen Typ

| | |
|---|---|
| Galbanum Öl ED | 0,5 |
| Eugenol | 1,0 |
| Methyljonon - gamma | 5,0 |
| Cis-3-Hexenylsalicylat | 3,0 |
| Benzylacetat | 8,0 |
| Brahmanol | 5,0 |
| Hedione (a) | 10,0 |
| Benzylsalicylat | 10,0 |
| Hexylzimtaldehyd | 10,0 |
| Phenylethylalkohol | 15,0 |
| Nerol | 10,0 |
| Bourbon Geranium Öl | 7,5 |
| Ylang-ylang Öl | 5,0 |
| Total | 90,0 |

Die Parfümbase der angegebenen Formel besitzt einen harmonischen, blumigen-grünen Duftcharakter.

Es wurden die beschriebenen Parfümbasen 8.1, 8.2 und 8.3 anschießend mit den oben beschriebenen Proben M 1, M 2 und M 3 (Beispiel 7) in den im folgenden beschriebenen Konzentrationen vermischt und anschließend durch das Expertenpanel aus acht Personen sensorisch beurteilt.

### (a) Mischungen mit Base 8.1:

| | (A) | (B) | (C) |
|---|---|---|---|
| Base 8.1 | 83 | 83 | 83 |
| M 1 | 17 | - | - |
| M2 | - | 17 | - |
| M3 | - | - | 17 |

Die hergestellten Mischungen wurden zunächst frisch und zur Beurteilung der Nachgerüche in Abständen von 8 und 24 Stunden sensorisch beurteilt. Dabei wiesen die Mischungen (A) und (C) einen unharmonischen, nicht abgerundeten, muffigen Gesamteindruck auf. Bei den Beispielen A und C ging der anfängliche blumig, holzige Duftcharakter verloren, die Geruchsintensität verlagerte sich dadurch in Geruchseindrücke mit naß pudrigen Anklängen. Diese an nasses Fell erinnernden Geruchstonalitäten werden allgemein als unangenehm empfunden. Der negative Gesamteindruck verstärkte sich insbesondere bei der Beurteilung der Nachgerüche der Mischungen (A) und (C), wobei die bei der Beurteilung der Reingerüche als negativ empfundenen schweißigen bzw. fekalischen Geruchseindrücke sich verstärkten.

Mischung (B) hingegen zeichnete sich nicht nur durch eine erhöhte Substantivität aus, d. h. sie verbleibt deutlich länger auf dem getrockneten Riechstreifen, sondern es verlagerte sich auch der blumig - holzige Geruchseindruck der Ursprungsbase zu einer hellen, leicht diffusen Ambra-Note mit deutlichen Anklängen an natürliches Ambra.

### (b) Mischungen mit Base 8.2

| | (D) | (E) | (F) |
|---|---|---|---|
| Base 8.2 | 83,5 | 83,5 | 83,5 |
| M 1 | 16,5 | - | - |
| M 2 | - | 16,5 | - |
| M 3 | - | | 16,5 |

Die Mischungen (D) und (F) zeichneten sich durch einen insgesamt vergleichbaren Geruchseindruck aus. Im Bereich der Kopfnote hingegen wird bei Mischung (D) eine deutlich störende, unharmonisch wirkende leicht süßlich-säuerlichen Fehlnote festgestellt. Diese lässt die Mischung unausgewogen und nicht so würzig erscheinen, wie das bei Basen diesen Typs erwünscht ist. Bei Mischung (F) wird im Bereich der Nachgerüche eine sich verstärkende Fehlnote festgestellt. Diese an nasses Leder erinnernde Fehlnote verstärkte sich mit zunehmender Trocknung. Der negative Geruchseindruck verstärkte sich in zusätzlichen Tests, wenn der Gewichtsanteil an Komponente M 3 in der Base erhöht wurde.

Mischung (E) hingegen besitzt harmonische, die Kopfnote betonende, in solchen Noten erwünschte Anklänge an Bergamotte und Muskat, wobei die sie verstärkenden würzigen Aspekte und Anklänge edler Holzarten den Gesamteindruck als sehr ausgewogen erscheinen lassen. Leicht säuerliche animalische Geruchseindrücke in Base 8.2 konnten durch Zumischen von M 2 überdeckt bzw. maskiert werden. M2 zeichnete sich nicht nur durch maskierende Effekte aus, auch Aufziehvermögen und Haftung wurden deutlich verbessert. Gleichzeitig wurde im Angeruch eine Hervorhebung der Kopfnote festgestellt.

### (c) Mischungen mit Base 8.3

Die Parfümbase aus Beispiel 9 wurde mit den Proben M 1, M 2 und M 3 in den angebebenen Gewichtsanteilen vermischt, die Mischungen anschließend separat in eine Seifenformulierung eingearbeitet und die resultierende Seife durch das Expertenpanel sensorisch beurteilt.

| | (G) | (H) | (J) |
|---|---|---|---|
| Parfümbase aus Beispiel 9 | 90,0 | 90,0 | 90,0 |
| M 1 | 10,0 | - | - |
| M 2 | - | 10,0 | - |
| M 3 | - | - | 10,0 |

Das Expertenpanel bevorzugte eindeutig Komposition (H), wobei sich diese durch eine insgesamt klarere Ausstrahlung, durch mehr Frische sowie einen sehr ausgewogenen harmonischen Geruchseindruck darstellte.

## Patentansprüche

1. Verfahren zur Herstellung von 1-(2,2,6-Trimethylcyclohexyl)-hexan-3-ol der FormelA 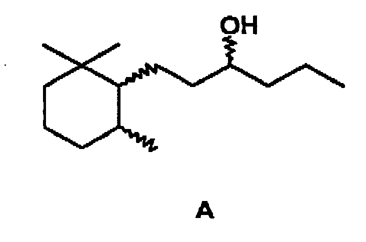 mit einem Anteil von zumindest 30 Gew.-% des entsprechenden trans-Isomeren, bezogen auf die Gesamtmenge 1-(2,2,6-Trimethylcyclohexyl)-hexen-3-ol der Formel A, mit folgendem Schritt:
- Katalytisches Hydrieren einer Verbindung der Formel D, 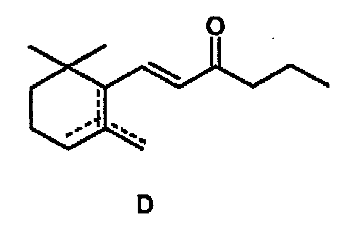 in der die gestrichelten Linien für eine einzelne Doppelbindung stehen, die in einer der drei eingezeichneten Positionen angeordnet ist, in Gegenwart eines Rhodium-Katalysators zum 1-(2,2,6-Trimethylcyclohexyl)-hexan-3-ol der Formel A,
wobei die Hydrierung in einem Wasserstoff-Druckbereich von 80 bis 200 bar, bevorzugt im Bereich von 80 bis 150 bar, durchgeführt wird,
wobei die katalytische Hydrierung bei einer Temperatur im Bereich von 180 bis 260 °C durchgeführt wird.

2. Verfahren nach Anspruch 1, wobei die Hydrierung erst nach Umsetzung von zumindest 98 % der Verbindung der Formel D beendet wird.

3. Verfahren nach einem der vorangehenden Ansprüche, wobei die Hydrierung in einem Wasserstoff-Druckbereich von über 100 bis maximal 200 bar, vorzugsweise im Bereich von über 100 bis maximal 150 bar, durchgeführt wird.

4. Verfahren nach einem der vorangehenden Ansprüche, wobei Rhodium in einer Menge von 0,0001 bis 10 Gew.-% eingesetzt wird, vorzugsweise im Bereich von 0,001 bis 0,5 Gew.-%, bezogen auf die Menge der eingesetzten Verbindung der Formel D.

5. Verfahren nach einem der vorangehenden Ansprüche, wobei
(a) das hergestellte 1-(2,2,6-Trimethylcyclohexyl)-hexan-3-ol der Formel A
(i) von dem Rhodium-Katalysator und/oder
(ii) von sonstigen Bestandteilen des Produktgemisches abgetrennt und/oder
(b) ein Isomeres des hergestellten 1-(2,2,6-Trimethylcyclohexyl)-hexan-3-ols der Formel A aus dem Produktgemisch abgetrennt oder aufkonzentriert wird.

6. Verfahren zur Herstellung einer Parfümkomposition, mit folgenden Schritten:
- Herstellen von 1-(2,2,6-Trimethylcyclohexyl)-hexan-3-ol gemäß einem Verfahren nach einem der vorangehenden Ansprüche,
- Gegebenenfalls Isolieren des 1-(2,2,6-Trimethylcyclohexyl)-hexan-3-ols aus dem Produktgemisch und/oder Reinigen des Produktgemisches,
und nachfolgend
- Vermischen einer sensorisch wirksamen Menge des 1-(2,2,6-Trimethylcyclohexyl)-hexan-3-ols mit einem oder mehreren weiteren Riechstoffen.

## Claims

1. Method for the manufacture of 1-(2,2,6-trimethyl cyclohexyl)-hexane-3-ol of formula A 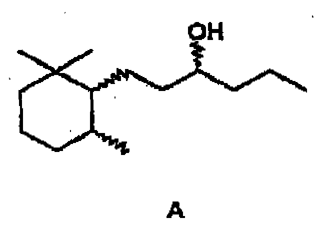 with a proportion of at least 30% by weight of the corresponding trans-isomers in relation to the total quantity of 1-(2,2,6-trimethyl cyclohexyl)-hexane-3-ol of formula A, with the following step:
- Catalytic hydration of a compound of formula D 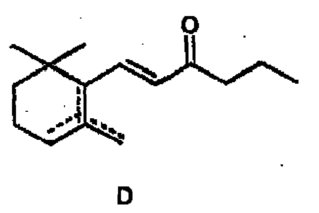
in which the broken lines stand for an individual double bond, which is arranged in one of the three positions drawn in, in the presence of a rhodium catalyst for the 1-(2,2,6-trimethyl cyclohexyl)-hexane-3-ol of formula A,
wherein the hydration is carried out in a hydrogen pressure range from 80 to 200 bar, preferably in the range from 80 to 150 bar,
wherein the catalytic hydration is carried out at a temperature in the range from 180 to 260 °C.

2. Method according to Claim 1, wherein the hydration is only completed after the conversion of at least 98 % of the compound from formula D.

3. Method according to any one of the preceding claims, wherein the hydration takes place in a hydrogen pressure range of above 100 to a maximum of 200 bar, preferably in the range from over 100 to a maximum of 150 bar.

4. Method according to any one of the preceding claims, wherein rhodium is introduced in a quantity from 0.0001 to 10% by weight, preferably in the range from 0.001 to 0.5% by weight, in relation to the quantity of the compound of formula D used.

5. Method according to any one of the preceding claims, wherein (a) the manufactured 1-(2,2,6-trimethyl cyclohexyl)-hexane-3-ol of formula A is separated (i) from the rhodium catalyst and/or (ii) from other constituents of the product mixture and/or (b) an isomer of the manufactured 1-(2,2,6-trimethyl cyclohexyl)-hexane-3-ol of formula A is separated or concentrated from the product mixture.

6. Method for the manufacture of a perfume composition, with the following steps:
- Manufacture of 1-(2,2,6-trimethyl cyclohexyl)-hexane-3-ol in accordance with a method according to any one of the preceding claims,
- As appropriate, isolation of the 1-(2,2,6-trimethyl cyclohexyl)-hexane-3-ol from the product mixture and/or cleaning of the product mixture
and subsequently
- Mixing of a sensorily efficacious quantity of the 1-(2,2,6-trimethyl cyclohexyl)-hexane-3-ol with one or more further aromatic substances.

## Revendications

1. Procédé de production du 1-(2,2,6-triméthylcyclohexyl)-hexan-3-ol de formule A 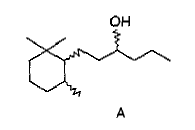 comportant une fraction d'au moins 30 % en masse de l'isomère trans correspondant, par rapport à la quantité totale de 1-(2,2,6-triméthylcyclohexyl)-hexan-3-ol de formule A, comportant l'étape suivante :
- hydrogénation catalytique d'un composé de formule D 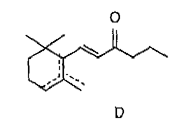
où les traits pointillés représentent une seule double liaison qui est disposée dans l'une des trois positions représentées, en présence d'un catalyseur au rhodium en le 1-(2,2,6-triméthylcyclohexyl)-hexan-3-ol de formule A,
où l'hydrogénation est conduite dans une plage de pression d'hydrogène de 80 à 200 bar, de préférence dans la plage de 80 à 150 bar,
où l'hydrogénation catalytique est conduite à une température dans la plage de 180 à 260°C.

2. Procédé selon la revendication 1 où l'hydrogénation n'est terminée qu'après la conversion d'au moins 98 % du composé de formule D.

3. Procédé selon l'une des revendications précédentes où l'hydrogénation est conduite dans une plage de pression d'hydrogène de plus de 100 à 200 bar au maximum, de préférence dans la plage de plus de 100 à 150 bar au maximum.

4. Procédé selon l'une des revendications précédentes où le rhodium est utilisé en une quantité de 0,0001 à 10 % en masse, de préférence dans la plage de 0,001 à 0,5 % en masse, par rapport à la quantité du composé de formule D utilisé.

5. Procédé selon l'une des revendications précédentes où
(a) le 1-(2,2,6-triméthylcyclohexyl)-hexan-3-ol de formule A produit est séparé
(i) du catalyseur au rhodium et/ou
(ii) d'autres constituants du mélange de produits et/ou
(b) un isomère du 1-(2,2,6-triméthylcylclohexyl)-hexan-3-ol de formule A produit est séparé du mélange de produits ou est concentré.

6. Procédé de production d'une composition de parfum comportant les étapes suivantes :
- production du 1-(2,2,6-triméthylcyclohexyl)-hexan-3-ol selon un procédé selon l'une des revendications précédentes,
- éventuellement isolement du 1-(2,2,6-triméthylcyclohexyl)-hexan-3-ol à partir du mélange de produits et/ou purification du mélange de produits,
puis
- mélange d'une quantité efficace du point de vue sensoriel du 1-(2,2,6-triméthylcyclohexyl)-hexan-3-ol avec une ou plusieurs autres substances odorantes.
